# EUROPEAN PATENT SPECIFICATION

(11) **EP 2 402 052 B1**
(45) Date of publication and mention of the grant of the patent: **06.03.2019**
(21) Application number: 11007963.9
(22) Date of filing: 02.06.2006
(51) Int. Cl.: A61M 25/10, A61M 39/24, A61M 39/02, A61B 17/12

(54) **Balloon catheter**
Ballonkatheter
Cathéter de Fogarty

(30) Priority: 02.06.2005 JP 2005162348
(43) Date of publication of application: 04.01.2012
(62) Divisional of application: 06756918.6
(73) Proprietor: Olympus Corporation, Tokyo 192-8507 (JP)
(72) Inventor: Nagamatsu, Ryuji, Hachioji-shi Tokyo 1920919 (JP); Yanuma, Yutaka, Tokyo 192-8507 (JP)
(74) Representative: Schicker, Silvia

(56) References cited:
- JP-A- 6 154 320
- JP-A- 2004 329 720
- US-A- 3 799 171
- US-A- 4 116 201
- US-A- 4 147 170
- US-A- 5 453 097
- US-A- 5 749 853
- US-A- 5 935 112

## Description

### BACKGROUND OF THE INVENTION

### Field of the Invention

The present invention relates to a balloon catheter that is used by being introduced into a body cavity.

### Description of Related Art

As a method of extracting stones in a common bile duct to the duodenum endoscopically, a method of arranging a balloon in a bile duct while performing X-ray imaging and then expanding the balloon at a high pressure to expand the bile duct opening to an extent that allows stones to be extracted has presently become widely prevalent. A balloon catheter as shown in Patent Document 1 (JP 2002-143311 A) is, for example, used for this method.

A syringe into which a fluid is inserted for expanding the balloon via an open-close valve is attached to the balloon catheter. For that reason, it is possible to expand the balloon by supplying the fluid from the syringe with the open-close valve opened, and after expanding the balloon, it is possible to maintain the expanded state of the balloon by preventing backflow of the fluid from the balloon by closing the open-close valve. Also, when contracting the balloon, it is possible to do so by opening the open-close valve to discharge the fluid from the balloon to the syringe.

In the above-mentioned conventional balloon catheter, when the operator performs the endoscope operation and balloon expansion operation, it is possible to proceed without assistance until the syringe operation. However, in order to maintain
the expansion of the balloon, the procedure of operating the open-close valve while pressing down the syringe cannot be performed alone, and so a coordinated operation with an assistant is required. For that reason, the expansion-and-contraction operation of the balloon cannot be performed up to the expectations of the operator. Accordingly, the procedure time increases, which leads to an increase in X-ray exposure.

The present invention was achieved in view of the above circumstances, and has as its object to provide a balloon catheter enabling an operator to perform expansion or contraction of the balloon without assistance and enabling a shortening of the procedure time by improving operability.

Document JP 06-154320 A (and its patent family member JP 3,102,173 B) may be construed to disclose a technique for liquid drug injection appliance, in which a flow rate control part to be used for this liquid drug injection appliance has a cylindrical body fixing the orifice body consisting of the deformable rubbery elastic body. A plug body is mounted on the upstream side of the liquid drug of this cylindrical body and a cap body is mounted on the downstream side of the liquid drug. The valve hole of the orifice body is provided with liquid drug tubes behind this valve hole in its axial direction. The valve hole is so shaped that its bore is in the middle entering from its inlet and expand gradually towards the downstream side from this position. The orifice body is pressed by rotating the plug body screwed with the cylindrical body, by which the bore of the valve hole is adjusted. A filter is interposed between the valve hole and the upstream side tube.

Document US 4,116,201 A may be construed to disclose a catheter comprising an elongated shaft, an inflatable balloon secured to the shaft, and an inflation lumen extending along the shaft and communicating with the balloon. The catheter has valve means communicating with the inflation lumen, with the valve means being normally closed and being openable to pump fluid into the inflation lumen for inflating the balloon. The catheter also has vent means communicating with the inflation lumen, with the vent means being normally closed and opening responsive to closure of the valve means and a pressure in the balloon above a predetermined amount, such that the vent means relieves pressure in the balloon above the predetermined amount after inflation of the balloon.

Document US 4,147,170 A may be construed to disclose a catheter comprising an elongated shaft, an inflatable balloon secured to the shaft, and an inflation lumen extending along the shaft and communicating with the balloon. The catheter has an inflation control assembly comprising a housing having a chamber, and a control member projecting into the chamber and having a circumferential sealing surface. The control assembly has a generally tubular valve element of flexible material
having a sealing portion biased against the sealing surface. The valve element normally prevents passage of fluid through the housing, and flexes away from the sealing surface to permit inflation of the balloon. The valve element also flexes away from the sealing surface at a predetermined pressure in order to relieve pressure in the inflated balloon when overinflated.

Document US 5,749,853 A may be construed to disclose a technique in which a time duration of the inflation and deflation cycles for a syringe assembly for an inflation system are measured and displayed. An inflation cycle is initiated when an increase-pressure switch is depressed. A timer will begin timing the inflation cycle when the increase-pressure switch is released and a predetermined initial pressure is detected in a tube of the syringe assembly. A deflation cycle is initiated when a decrease-pressure switch is depressed. The timer will stop measuring the inflation cycle and then begin timing the deflation cycle when the decrease-pressure switch is released and a predetermined neutral pressure is detected in a tube of the syringe assembly. Rapid deflation can be achieved by depressing a rapid-decrease-pressure switch. Upon actuation of the rapid-decrease-pressure switch, the timer immediately stops measuring the inflation cycle and begins timing the deflation cycle.

Document US 3,799,171 A may be construed to disclose an inflation valve for a retention balloon in a catheter having an inflation lumen communicating with the balloon. The valve has a housing defining a chamber, opening means at one end of the housing communicating between the chamber and the inflation lumen, and an inwardly directed flange at the other end of the housing. A rim is secured to the flange and has a first portion projecting into the chamber, a second portion projecting outwardly from the flange, and a tapered passageway extending through the rim. The valve also includes a compressible plug received in the chamber and having a first shoulder, a neck extending from the first shoulder, a second shoulder extending from the neck, and a tapered stopper extending from the second shoulder and received in the passageway. The plug biases the stopper against the tapered portion of the passageway in sealing engagement to prevent passage of fluid from the chamber, and the plug compresses responsive to the insertion of a syringe tip into the passageway from its outer end to permit passage of fluid from the syringe into the chamber and inflation lumen.

### SUMMARY OF THE INVENTION

The present invention adopts the following constitutions in order to solve the aforesaid problems.

According to the disclosure, there is provided an apparatus according to the independent claim. Developments are set forth in the dependent claims.

A balloon catheter preferably is provided with a catheter shaft having flexibility with a lumen formed inside; a balloon that is disposed at the distal end side of the catheter shaft and in communication with the lumen; a fluid regulating portion that passes a fluid that is supplied from a fluid supply source to the balloon and, simultaneous with the supply from the fluid supply source being stopped, prevents backflow of the fluid that is supplied to the balloon to the fluid supply source side; and a fluid releasing portion that releases the fluid that is supplied to the balloon to outside of the balloon.

This balloon catheter preferably supplies a fluid from a fluid supply source to a balloon to expand the balloon and, simultaneous with stopping the fluid supply, can maintain the expansion of the balloon by confining the fluid in the balloon. Also, it is preferably possible to contract the balloon by releasing the fluid to outside of the balloon by the fluid releasing
portion.

Also, in the case of independently arranging a supply operating member, a fluid regulating portion, and a fluid operating portion from the fluid supply source within the range that the operator can control with one hand, when the operator performs with one hand the fluid supply operation from the fluid supply source to the balloon, it is possible to perform expansion of the balloon and maintenance of the expansion with one hand. In addition, it is possible to contract the balloon by only a fluid releasing operation with one hand by the fluid releasing portion.

The balloon catheter preferably further comprises an operating portion that has the fluid regulating portion and the fluid releasing portion; with a fixing portion that detachably mounts the operating portion on an endoscope operating portion of an endoscope or vicinity thereof is provided at the operating portion.

With this balloon catheter, the operator controlling the endoscope can perform not only the expansion and maintenance operations of a balloon but also the balloon contraction operation in a stable state in the case of performing the fluid supply operation from the fluid supply source.

The fluid releasing portion is preferably disposed more to the balloon side than the fluid regulating portion.

With this balloon catheter, when releasing the fluid from the balloon to the outside, it is possible to discharge the fluid from the fluid releasing portion to the outside before the fluid reaches the fluid releasing portion. Accordingly, when the operator performs fluid supply alone, it is possible to smoothly perform contraction of the balloon alone.

The balloon catheter preferably further comprises a valve portion in which is disposed a connection opening that is connected with the base end side of the catheter shaft, an attachment opening that is connected with the fluid supply source that supplies fluid for expanding the balloon, and a release opening to the atmosphere; wherein the fluid releasing portion is disposed in the valve portion as a plug portion that selects either one of a first pipeline that allows communication between the connection opening and the attachment opening and a second pipeline that branches from the first pipeline and allows communication between the connection opening and the release opening; and the fluid regulating portion is disposed in the first pipeline between the position where the plug portion is disposed and the attachment opening.

With this balloon catheter, when supplying fluid from the fluid supply source in the state of the fluid supply source being connected to the attachment opening of the valve portion, the catheter shaft being connected to the connection opening, and the first pipeline allowing communication between the connection opening and the attachment opening, it is possible to expand the balloon by supplying the fluid to the lumen of the catheter shaft.

At this time, since the check valve is disposed in the valve portion, when maintaining the expansion of the balloon, even without particularly performing the operation of the fluid supply source and the balloon catheter, it is possible to favorably prevent backflow of the supplied fluid to the fluid supply source side, and possible to maintain the expanded state of the balloon, and possible to perform expansion of the balloon and maintenance thereof with the operation of one hand.

Also, since the check valve is disposed between the attachment opening and the plug portion, when contracting the balloon, by switching the plug portion to allow communication between the connection opening and the release opening, it is possible to discharge the fluid from the discharge opening to the outside with an operation by one hand only. Therefore, it is possible to perform the series of operation of expanding a balloon, maintenance thereof, and contraction with one hand and therefore possible for an operator to operate the balloon catheter alone.

The balloon catheter preferably further comprises a catheter shaft having flexibility with a lumen formed inside; a balloon that is disposed at the distal end side of the catheter shaft and in communication with the lumen; and a valve portion in which is disposed a connection opening that is connected with the base end side of the catheter shaft, and an attachment opening that is connected with a fluid supply source that supplies fluid for expanding the balloon; wherein the valve portion is provided with a valve body in which is disposed a pipeline that allows communication between the connection opening and the attachment opening; a plug portion that is disposed in the pipeline and that elastically deforms to shut the pipeline; and a press operation portion that presses and deforms the plug portion.

This balloon catheter can supply the fluid to the lumen of the catheter shaft to expand the balloon by supplying the fluid from the fluid supply source by connecting the fluid supply source to the attachment opening of the valve portion and connecting the catheter shaft to the connection opening.

At this time, by operating the press operation portion to press the plug portion, the plug portion elastically deforms so that the space between the connection opening and the attachment opening is blocked. Accordingly, backflow of the fluid that has flowed into the balloon is prevented, so that it is possible to maintain the expansion of the balloon. When contracting the balloon, by operating the press operating portion to release the deformation of the plug portion and allow communication between the
connection opening and the attachment opening, it is possible to discharge fluid from the balloon.

Preferably, one of a screw portion and a thread portion is disposed on the valve body while the other is disposed on the press operation portion, so that the valve body and the press operation portion can be screwed together; and a regulating portion that regulates co-rotation of the valve body when screwing the press operation portion with respect to the valve body is disposed on the catheter shaft.

With this balloon catheter, when screwing the valve body and the press operation body, by adjusting the screw state, it is possible to adjust the deformation state of the screw portion. When doing so, since the regulating portion is disposed on the catheter shaft, even when the press operation portion is rotated, it is possible to regulate the rotation of the valve body that is connected to the catheter shaft, and it is possible to reliably rotate the press operation portion with respect to the valve body.

The the attachment opening is preferably disposed in the press operation portion.

With this balloon catheter, by attaching the fluid supply source to the attachment opening of the valve opening and connecting the fluid supply source and the press operation portion, it is possible to rotate the fluid supply source in the screw direction to rotate the press operation portion. Accordingly, by facilitating the rotation of the press operation portion, the operability can be improved.

The balloon catheter preferably further comprises an operating portion that has the valve portion, with a fixing portion that detachably mounts the operating portion on an endoscope operating portion or vicinity thereof of an endoscope provided at the operating portion.

With this balloon catheter, the operator operating the endoscope can perform not only the expansion and maintenance operations of a balloon but also the balloon contraction operation in a stable state in the case of performing the fluid supply operation from the fluid supply source.

According to the present disclosure, it is possible for an operator to perform the operations of expanding a balloon, maintenance thereof, and contraction alone without requiring the help of an assistant, and possible to shorten the procedure time by improving the balloon operability.

### BRIEF DESCRIPTION OF THE DRAWINGS

FIG 1 is a view showing the overall outline of the balloon catheter according to an example not forming part of the present invention.
FIG. 2A is a plan view showing the state when expanding the balloon of the valve portion of the balloon catheter according to the example and when maintaining the expansion.
FIG. 2B is a plan view showing the state when contracting the balloon of the valve portion of the balloon catheter according to the example.
FIG 3A is a left elevation view showing the state of the check valve that is disposed in the valve portion of the balloon catheter according to the example stopping the flow of air.
FIG. 3B is a side cross-sectional view showing the state of the check valve that is disposed in the valve portion of the balloon catheter according to the example stopping the flow of air.
FIG. 3C is a right elevation view showing the state of the check valve that is disposed in the valve portion of the balloon catheter according to the example stopping the flow of air.
FIG. 3D is a side cross-sectional view showing the state of the check valve that is disposed in the valve portion of the balloon catheter according to the example passing air.
FIG. 4 is a view showing the overall outline of the balloon catheter according to the embodiment of the present invention.
FIG. 5A is a side cross-sectional view showing the state when expanding the balloon of the valve portion of the balloon catheter according to the embodiment of the present invention and when maintaining the expansion.
FIG. 5B is a side cross-sectional view showing the state when contracting the balloon of the valve portion of the balloon catheter according to the embodiment of the present invention.
FIG. 6 is a cross-sectional view along line B-B in FIG. 4
FIG. 7A is a side cross-sectional view showing the state of the check valve in another example of the check valve stopping the flow of air.
FIG. 7B is a side cross-sectional view showing the state of the check valve in another example of the check valve passing air.

### DETAILED DESCRIPTION OF THE INVENTION

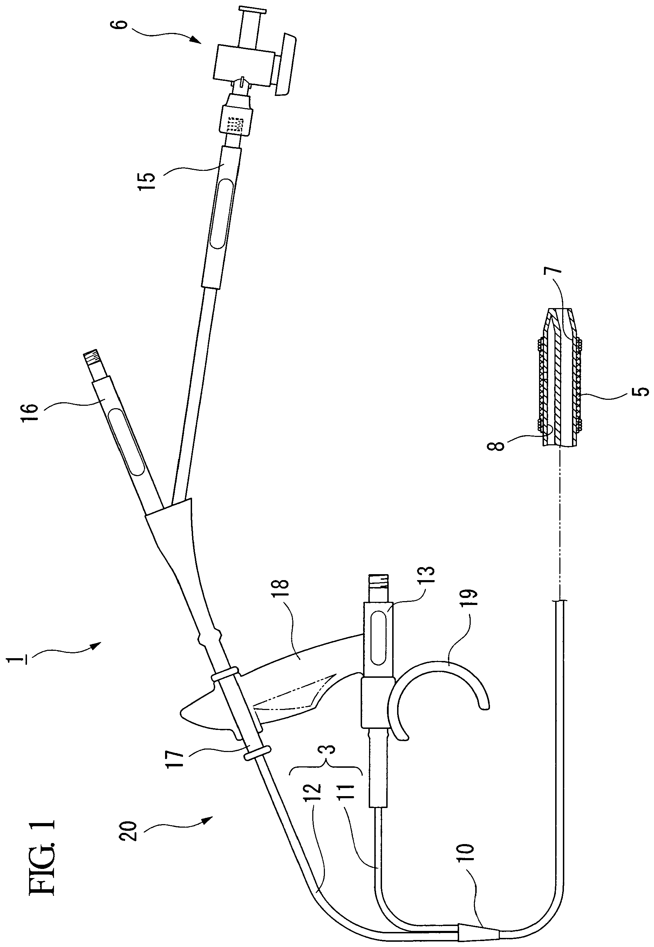

The first example not forming part of the invention shall be described with reference to FIG. 1 to FIG. 3D.

A balloon catheter 1 is as shown in FIG. 1 able to advance and retreat in a channel of an endoscope not illustrated along a guide wire not illustrated. This balloon catheter 1 is provided with a catheter shaft 3 that is narrow and flexible, a balloon 5 that is disposed on the distal end side of the catheter shaft 3, and a valve portion 6 that is connected with the base end side of the catheter shaft 3.

The catheter shaft 3 is formed from a guide wire lumen 7 that is flexible and through which a guide wire is passed, a balloon lumen (lumen) 8 through that is made continuous with the balloon 5 and through which air (fluid) is passed for expanding and contracting the balloon 5, and a contrast agent lumen not illustrated through which a contrast agent is passed.

The catheter shaft 3 moreover is branched by a branching portion 10 disposed on the base end side into a first shaft 11 in which the guide wire lumen 7 is arranged and a second shaft 12 in which the balloon lumen 8 and the contrast agent lumen are arranged.

A wire mouthpiece 13 for passing the guide wire is disposed at the base end of the first shaft 11.

The base end side of the second shaft 12 further branches into two, with a valve mouthpiece 15 that is connected to the valve portion 6, and a contrast agent mouthpiece 16 for flowing in the contrast agent being disposed.

On the wire mouthpiece 13 is disposed a support portion 18 that can detachably support a connector 17 that is arranged on the second shaft 12 for positioning the second shaft 12 with respect to the first shaft 11 at a predetermined angle and spacing.

A fixing portion 19 that is formed in a substantial C-shape to be engageable with the operation portion not illustrated on the endoscope or thereabouts is disposed on the support portion 18 for positioning the wire mouthpiece 13 with respect to the endoscope or the like.

Note that the balloon catheter 1 is provided with a total operating portion (operating portion) 20 that is constituted by the members from the branching portion 10 to the valve portion 6 on the proximal side thereof and the contrast agent mouthpiece 16.

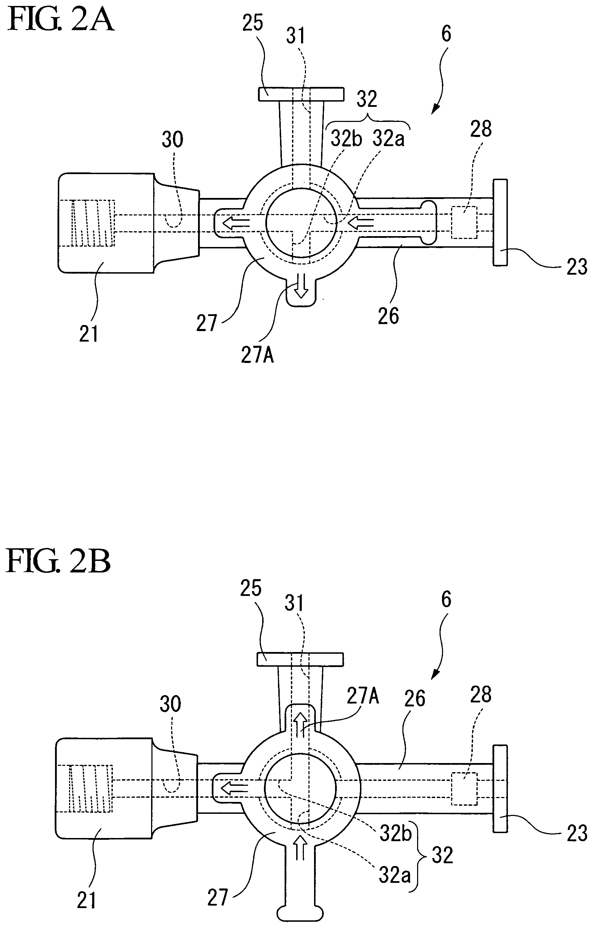

The valve portion 6 is, as shown in FIG. 2A and FIG. 2B, provided with a valve body 26 in which is disposed a connection opening 21 that is connected to a valve mouthpiece 15 of the second shaft 12, an attachment opening 23 that is connected to a syringe (fluid supply source) not illustrated that supplies air for expanding the balloon 5, and a release opening 25 to the atmosphere; a plug portion (fluid releasing portion) 27 that selects either one of a first pipeline 30 and a second pipeline 31 and thus makes continuous the connection opening 21 and the release opening 25, or the attachment opening 23 and the release opening 25; and a check valve (fluid regulating portion) 28 that is disposed in the first pipeline 30 between the plug portion 27 and the attachment opening 23 and allows the passage of fluid heading from the attachment opening 23 side to the connection opening 21 side while blocking fluid heading from the connection opening 21 side or opening 25 side to the attachment opening 23 side.

At the valve body 26 is disposed the first pipeline 30 that allows communication between the connection opening 21 and the attachment opening 23 and the second pipeline 31 that branches from the first pipe 30 and allows communication between the connection opening 21 and the release opening 25. In the plug portion 27 is disposed a T-pipeline 32 that consists of a main pipe 32a and a support pipe 32b that is connected to the middle of the main pipe 32a to communicate therewith and constitute a T-tube. On the surface of the plug portion 27 is provided an indicator portion 27A for alignment of the first pipeline 30 and the second pipeline 31 with both end positions of the main pipe 32a and the end portion position of the support pipe 32b.

When the plug portion 27 is arranged in the position shown in FIG. 2A, the T-pipeline 32 puts the connection opening 21 and the attachment opening 23 in a state of communication since the first pipeline 30 and the main pipe 32a are in communication. On the other hand, since the end portion of the support pipe 32b and the second pipeline 31 are facing different directions, the T-pipeline 32 blocks the opening 21 and the release opening 25. Also, when the plug portion 27 is arranged at the position shown in FIG. 2B, the end portion of the support pipe 32b faces the connection opening 21 side of the first pipeline 30, and one end of the main pipe 32a faces the release opening 25 side of the second pipeline 31. Thereby, while the connection opening 21 and the release opening 25 are put in a state of communication, the other end of the main pipe 32a is blocked off so that the connection opening 21 and the attachment opening 23 are blocked.

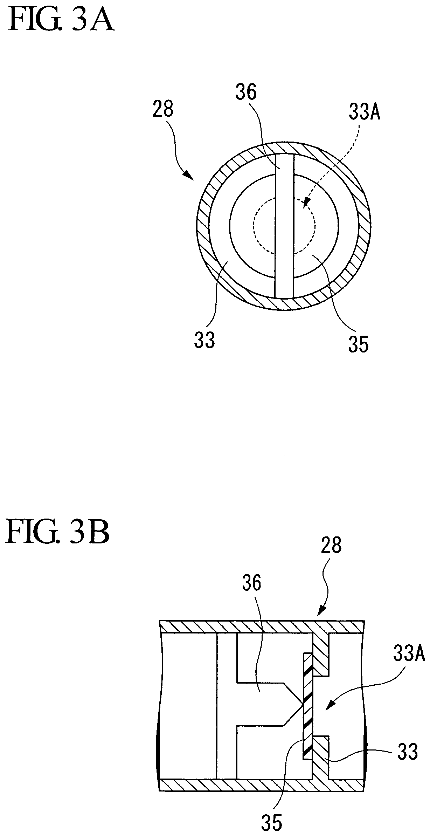

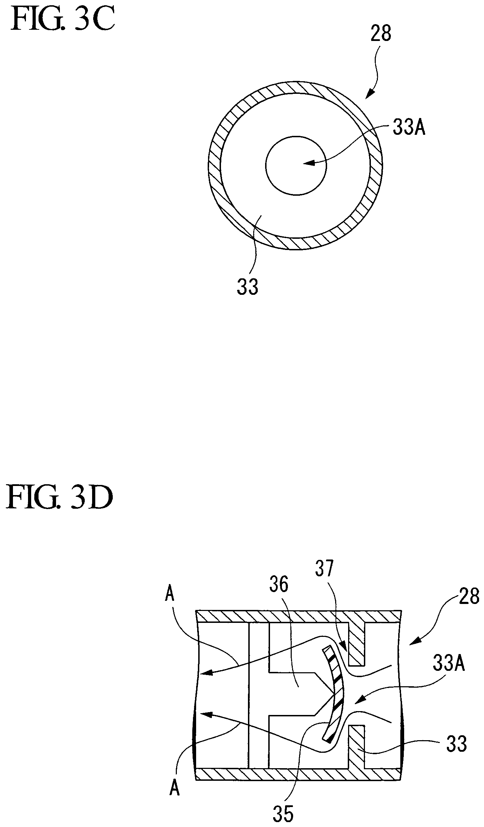

The check valve 28 is as shown in FIG. 3A, FIG. 3B, and FIG. 3C provided with an orifice portion 33 that is disposed projecting inward in the diameter direction of the first pipeline 30 and with an orifice hole 33A formed in the center thereof, a lid portion 35 that is constituted with a flexible material such as silicon and has a larger diameter than the orifice hole 33A so that the edge portion is in contact with the orifice portion 33, and a pressing portion 36 that presses at a predetermined pressure from a predetermined position the center portion of the lid portion 35 toward the attachment opening 23 side.

In this check valve 28, as shown in FIG. 3B, the orifice hole 33A is plugged as a result of the lid portion 35 being pressed on the orifice portion 33 by air when air is flowing from the connection opening 21 side to the attachment opening 23 side. On the other hand, when air flows from the attachment opening 23 side to the connection opening 21 side, as shown in FIG. 3D, the lid portion 35 moves so as to separate from the orifice portion 33. However, because the movement of the lid portion 35 is restricted by the pressing portion 36, the lid portion 35 curves. At this time, a gap 37 is formed between the edge portion of the lid portion 35 and the orifice portion 33. Accordingly, air flows via the gap 37 as shown by the arrows A in the drawing.

Next, the method of operating the balloon catheter 1 being an example and not forming part of the present invention and the operation/effect shall be described.

First, an endoscope not illustrated is inserted into a body cavity, and a guide wire not illustrated is inserted until a predetermined position in a bile duct via the endoscope by a widely known method and operation, and a predetermined treatment is completed by a predetermined treatment tool.

Next, the balloon catheter 1 is prepared by connecting the connection opening 21 of the valve portion 6 to the valve mouthpiece 15 that is disposed on the second shaft 12 and connecting the syringe 52 to the attachment opening 23 of the valve portion 6.

Then, the distal end side of the catheter shaft 3 is inserted into the bile duct via the endoscope while inserting the guide wire into the guide wire lumen 7 of the balloon catheter 1, and the fixing portion 19 of the balloon catheter 1 is fixed to a predetermined position.

Also, by flowing the contrast agent into the contrast agent lumen not illustrated from the contrast agent mouthpiece 16, the treatment proceeds while performing X-ray imaging.

In the case of expanding the balloon 5, the operator, while grasping the endoscope with one hand throughout, turns the plug portion 27 of the valve portion 6 with the other hand to adjust the direction of the T-pipeline 32 so as to put the connection opening 21 and the attachment opening 23 in a state of communication. Thereby, the syringe 52 and the balloon lumen 8 communicate.

In this state, the syringe 52 is operated with the other hand to flow a predetermined quantity of air into the valve portion 6. At this time, due to the air pressure, the lid portion 35 of the check valve 28 tries to move in the direction to separate from the orifice portion 33. However, because the center portion abuts the pressing portion 36, the edge portion side curves, and the gap 37 is formed between the edge portion of the lid portion 35 and the orifice portion 33. The air flows then through the first pipeline 30 via this gap 37. Thereby, the air flows into the balloon 5, causing it to expand.

When the predetermined quantity of air has flowed, the pressure on the side of the balloon 5 rises instead of the air pressure from the syringe 52 decreasing. Due to this air pressure, the lid portion 35 is pressed onto the orifice portion 33, and so the orifice hole 33A is plugged. For this reason, although the air attempts to flow back to the syringe 52 side it is blocked and so the pressure in the balloon 5 is maintained so that its expansion state is maintained.

In this state, stones and the like are cleared away from within the bile duct and discharged therefrom.

When contracting the balloon 5, the operator, while grasping the endoscope with one hand throughout as described above, turns the plug portion 27 of the valve portion 6 with the other hand to adjust the direction of the T-pipeline 32 so as to put the connection opening 21 and the release opening 25 in a state of communication as shown in FIG. 2B. Thereby, the air in the balloon 5 and the balloon lumen 8 is discharged to the atmosphere from the release opening 25. Accompanying this, the balloon 5 contracts.

Thus, by removing the balloon catheter 1, or the entire endoscope, from the body cavity, the treatment by the balloon 5 is completed.

According to the balloon catheter 1, since the check valve 28 is disposed in the valve portion 6, when maintaining the expansion of the balloon 5, it is possible to suitably prevent the supplied air from flowing back to the syringe 52 side and maintain the expanded state of the balloon 5 without particularly performing operation of the syringe 52 or the balloon catheter 1.

Also, since the check valve 28 is arranged in the first pipeline 30 between the attachment opening 23 and the plug portion 27, when contracting the balloon 5, it is possible to discharge the air from the release opening 25 to the outside via the second pipeline 31 just by the operation of bringing the connection opening 21 and the release opening 25 into communication by switching the plug portion 27.

Accordingly, it is possible for an operator to perform the operations of expanding a balloon, maintenance thereof, and contraction with one hand, and so possible to perform work without requiring the help of an assistant.

For that reason, it is possible to ease the stress on the operator and cut down on X-ray exposure by shortening the procedure time, and also possible to realize a reduction in labor and other costs.

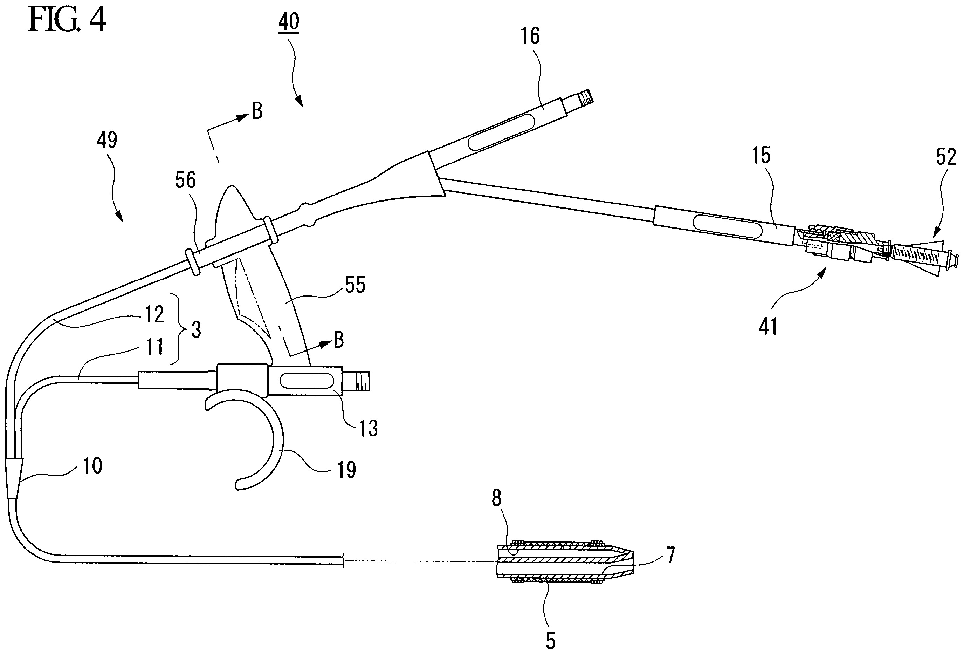

Next, an embodiment shall be described with reference to FIG. 4 to FIG. 6.

Note that constituent elements that are similar to those of the first example described above shall be assigned the same reference numerals and a detailed explanation thereof shall be omitted.

The point of difference between the embodiment and the first example is that a valve portion 41 of a balloon catheter 40 according to the present embodiment is provided with a valve body 46 in which is disposed a pipeline 45 that brings a connection opening 42 and an attachment opening 43 into communication, a plug portion 47 that is disposed in the pipeline 45 and elastically deforms to shut off the pipeline 45, and a press operation portion 48 that presses and deforms the plug portion 47.

Note that the balloon catheter 40 is provided with a total operating portion 49 that is constituted by the members from the branching portion 10 to the valve portion 41 on the proximal side thereof and the contrast agent mouthpiece 16.

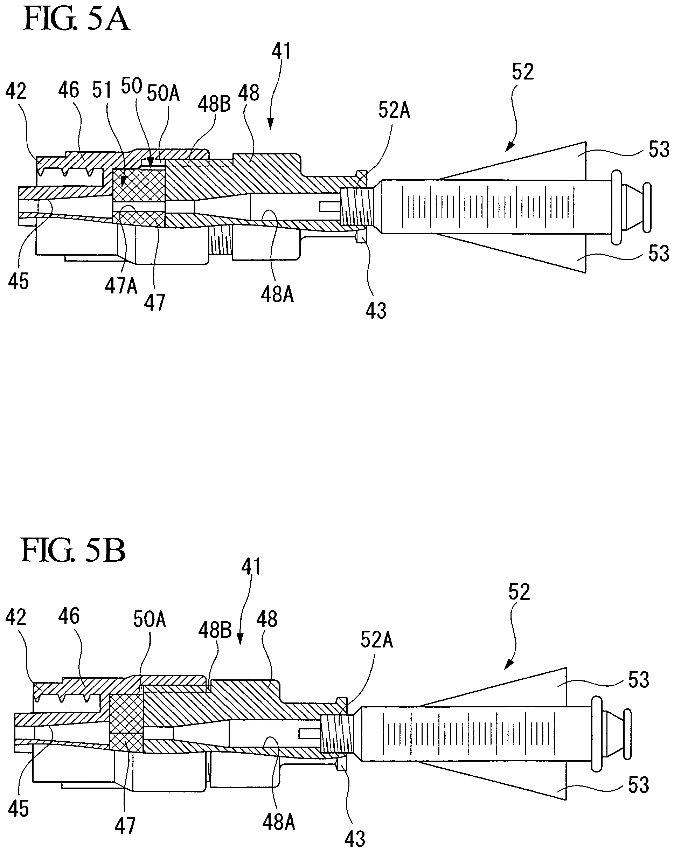

As shown in FIG. 4, FIG. 5A, and FIG. 5B, the connection opening 42 is disposed opening to the distal end of the valve body 46. A large diameter recess portion 50 in which a thread portion 50A is formed on the inside surface and a small diameter recess portion 51 that is formed further recessed from the bottom portion of the large diameter recess portion 50 are disposed on the base end side of the valve body 46.

The plug portion 47 is made from a rubber material with a through hole 47A formed with a smaller diameter than the pipeline 45 and in communication with therewith, being disposed fitted with the small diameter recess portion 51.

In the press operation portion 48, another through hole 48A that communicates with the pipeline 45 of the valve body 46 is disposed, and an attachment opening 43 is disposed at the end portion of this through hole 48A. At the distal end side face of this press operation portion 48, a screw portion 48B that screws with the thread portion 50A of the large diameter recess portion 50 is disposed.

Another thread portion 43A is formed in the inner surface of the attachment opening 43 that is capable of screwing with another screw portion 52A that is formed in the distal end of the syringe 52. Note that the screw direction of the syringe 52 and the press operation portion 48 is the same direction as the screw direction of the press operation portion 48 and the valve body 46.

Flaps 53 that project outward in the radial direction from the side surfaces of the syringe 52 are disposed in order to simplify the screwing with the press operation portion 48.

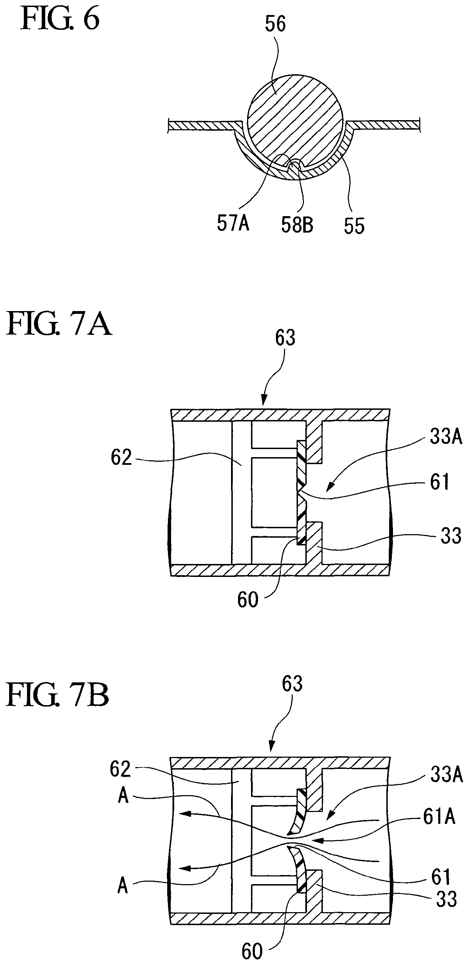

A regulating portion 57 that regulates the co-rotation of the valve body 46 when screwing the press operation portion 48 with respect to the valve body 46 is disposed on a support portion 55 that is arranged on the first shaft 11 and a connector portion 56 that is disposed on the second shaft 12. This restriction portion 57 is, as shown in FIG. 6, provided with a protruding portion 57A that is disposed on the support portion 55 and a recess portion 57B that is disposed on the connector portion 56 and engages with the protruding portion 57A.

Next, the method of operating the balloon catheter 40 according to the present embodiment and the operation/effect shall be described.

First, similarly to the first embodiment, a predetermined treatment is completed by a predetermined treatment device.

The balloon catheter 40 is prepared by screwing the press operation portion 48 into which the syringe 52 is screwed into the valve body 46 until making contact with the plug portion 47 that is disposed in the valve body 46, so that the connection opening 42 of the valve portion 41 and the valve mouthpiece 15 that is disposed on the second shaft 12 are connected.

Then, the distal end side of the catheter shaft 3 is inserted into the bile duct via the endoscope while inserting the guide wire into the guide wire lumen 7 of the balloon catheter 40, and the fixing portion 19 of the balloon catheter 40 is fixed to a predetermined position.

Also, the contrast agent is flowed into the contrast agent lumen not illustrated from the contrast agent mouthpiece 16 and the treatment proceeds while performing X-ray imaging.

In the case of expanding the balloon 5, the syringe 52 is operated to flow a predetermined quantity of air into the valve portion 41. At this time, since the plug portion is not elastically deformed, the connection opening 42 and the attachment opening 43 communicate. Accordingly, the air from the syringe 52 flows into the pipeline 45. In this way, the air flows into the balloon 5 via the balloon lumen 8, causing it to expand.

After completing the air supply from the syringe 52, the flaps 53 of the syringe 52 are immediately grasped and rotated in a direction to screw the syringe 52 into the press operation portion 48 from the state shown in FIG. 5A. At this time, as shown in FIG. 5B, the plug portion 47 is pressed by the press operation portion 48 and elastically deforms within the small diameter recess portion 51, so that the through hole 47A of the plug 47 is crushed. For that reason, the space between the connection opening 42 and the attachment opening 43 is blocked so that the pressure in the balloon 5 is maintained, and the expanded shape is maintained.

In this state, stones and the like are cleared away from within the bile duct and discharged therefrom.

When contracting the balloon 5, the flaps 53 of the syringe 52 or the press operation portion 48 is directly grasped, and the syringe 52 is rotated in a direction to be removed. Thereby, the pressing force on the plug portion 47 decreases, and the elastic deformation of the plug portion 47 returns to the original state, and the size of the through hole 47A of the plug portion 47 returns to the original shape. Thereby, the air in the balloon 5 and the balloon lumen 8 flows back into the syringe 52 from the pipeline 45, and the balloon 5 contracts.

Thus, by removing the balloon catheter 40, or the entire endoscope, from the body cavity, the treatment by the balloon 5 is completed.

According to this balloon catheter 40, by grasping and rotating the flaps 53 after the air inflow by the syringe 52, it is possible to maintain the expanded state of the balloon 5. Accordingly, it is possible to perform the operations of expanding a balloon, maintenance thereof, and contraction with one hand, and thus possible to perform a maneuver up to the expectations of the operator without requiring a coordinated operation between the operator and an assistant.

In particular, when screwing together the valve body 46 and the press operation portion 48, it is possible to finely adjust the deformation state of the plug portion 47 by adjusting the screw state. Thereby, during contraction of the balloon 5, the flow quantity of the fluid can be made a very small quantity, and thus it is possible to slowly contract the balloon 5. Also, since it is possible to finely adjust the outer diameter of the balloon 5, when removing a stone to the duodenum in the state of having expanded the balloon 5, as the diameter of the bile duct becomes narrower heading toward the downstream side, the outer diameter of the balloon 5 can be made smaller. Moreover, sine the restriction portion 57 is disposed on the connector portion and the support portion 55, even when the syringe 52 and the press operation portion 48 are rotated, it is possible to restrict rotation of the valve body 46 that is connected to the second shaft 12, and so it is possible to reliably rotate the press operation portion 48 with respect to the valve body 46.

Also, by grasping the flaps 53 and rotating the syringe 52 that is connected to the attachment opening 43 of the valve portion 41, it is possible to rotate the press operation portion 48 together, and possible to elastically deform the plug portion 47. In this case, since the flaps 53 are disposed outward in the radial direction beyond the outer radius of the press operation portion 48, it is possible to reduce the rotative force that is required, and so possible to improve the operability by simplifying the rotation of the press operation part 48.

Note that the technical scope of the present invention is not restricted to the preferred embodiments described above. Various modifications can be made without departing from the scope of the appended claims.

For example, in the first example the pressing portion 36 of the check valve 28 presses the edge portion of the lid portion 35 onto the orifice portion 33. However, as shown in the example of FIG. 7A, it is also possible to constitute a check valve 63 in which a cutout
portion 61 is disposed in the center portion of the lid 60, with a pressing portion 62 pressing the edge portion of the lid portion 35 onto the orifice portion 33.

By forming the shape of the cutout portion 61 to become wider from the connection opening side to the attachment opening side, as shown in the example FIG. 7B, when flowing in air from the syringe, the cutout portion 61 opens so that a passage hole 61A for the air is formed. Accordingly, air flows via this passage hole 61A in the directions shown by the arrows A in the drawing, and so it is possible to supply air to the balloon. Also, in the case of the air pressure on the connection opening side being high, the cutaway portion 61 is plugged and backflow of air is prevented, so that it is possible to maintain the expansion of the balloon.

### INDUSTRIAL APPLICABILITY

The present invention can be used for a device that is introduced to a body cavity of a living body, and a system that includes such a device.

## Claims

1. A balloon catheter (40) comprising
a catheter shaft (3) having flexibility with a lumen (8) formed inside;
a balloon (5) that is disposed at the distal end side of the catheter shaft (3) and in communication with the lumen (8); and
a valve portion (41) in which is disposed a connection opening (42) that is connected with the base end side of the catheter shaft (3), and an attachment opening (43) that is connected with a fluid supply source (52) configured to supply fluid for expanding the balloon (5),
wherein
the valve portion (41) is provided with a valve body (46) in which is disposed a pipeline (45) configured to allow communication between the catheter shaft (3) and the fluid supply source (52), a plug portion (47) that is disposed in the pipeline (45) and that is configured to communicate to the pipeline (45); and a press operation portion (48) that is disposed in a base end of the plug portion (47) and that is configured to press and deform the plug portion (47);
the attachment opening (43) is disposed at an end portion of a first through hole (48A) in the press operation portion (48); the connection opening (42) and the attachment opening (43) are arranged to communicate by screwing the press operation portion (48) into which the fluid supply source (52) is screwed into the valve body (46) until making contact with the plug portion (47) disposed on the valve body (46);
a thread portion (43A) formed in an inner surface of the attachment opening (43) is capable of screwing with another screw portion (52A) that is formed in a distal end of the fluid supply source (52), wherein the screw direction of the fluid supply source (52) and the press operation portion (48) is the same direction as the screw direction of the press operation portion (48) and the valve body (46); and
the plug portion (47) is arranged to be pressable by the press operation portion (48) to elastically deform within a base end side of valve body (46) so that a second through hole (47A) of the plug portion (47) is crushed.

2. The balloon catheter (40) according to Claim 1, wherein
a small recess portion (51) is disposed on the base end side of valve body (46), and
the plug portion (47) is arranged to be pressable by the press operation portion (48) to elastically deform within the small recess portion (51).

3. The balloon catheter (40) according to Claim 2, wherein
a large diameter recess portion (50) is arranged on the base end side of valve body (46), and
the small recess portion is formed further recessed from the bottom portion of the large diameter recess portion (50).

4. The balloon catheter (40) according to Claim 1,
further comprising a regulating portion (57) configured to regulate co-rotation of the valve body (46) when screwing the press operation portion (48) with respect to the valve body (46) is disposed on the catheter shaft (3).

5. The balloon catheter (40) according to Claim 1,
further provided with an operating portion (49) that has the valve portion (41);
wherein a fixing portion configured to detachably mount the operating portion on an endoscope operating portion or vicinity thereof of an endoscope is provided at the operating portion (49).

## Patentansprüche

1. Ballonkatheter (40), umfassend:
einen Katheterschaft (3), der Flexibilität hat und ein im Inneren gebildetes Lumen (8) aufweist;
einen Ballon (5), der an der Distalendseite des Katheterschafts (3) angeordnet ist und mit dem Lumen (8) in Verbindung steht; und
einen Ventilabschnitt (41), in welchem eine Verbindungsöffnung (42), die mit der Basisendseite des Katheterschafts (3) verbunden ist, und eine Befestigungsöffnung (43), die mit einer Fluidversorgungsquelle (52) verbunden ist, die dazu ausgestaltet ist, ein Fluid zum Ausdehnen des Ballons (5) zu liefern, angeordnet ist,
wobei
der Ventilabschnitt (41) mit einem Ventilkörper (46) versehen ist, in welchem ein Rohr (45), das dazu ausgestaltet ist, eine Verbindung zwischen dem Katheterschaft (3) und der Fluidversorgungsquelle (52) zu ermöglichen, ein Stopfenabschnitt (47), der in dem Rohr (45) angeordnet ist und dazu ausgestaltet ist, mit dem Rohr (45) in Verbindung zu stehen; und ein Druckbedienabschnitt (48), der in einem Basisende des Stopfenabschnitts (47) angeordnet ist und dazu ausgestaltet ist, den Stopfenabschnitt (47) zu drücken und zu verformen, angeordnet ist;
die Befestigungsöffnung (43) an einem Endabschnitt einer ersten Durchtrittsöffnung (48A) in dem Druckbedienabschnitt (48) angeordnet ist;
die Verbindungsöffnung (42) und die Befestigungsöffnung (43) so gestaltet sind, dass sie durch Schrauben des Druckbedienabschnitts (48), in welchen die Fluidversorgungsquelle (52) geschraubt ist, in den Ventilkörper (46) bis zur Kontaktaufnahme mit dem Stopfenabschnitt (47), der an dem Ventilkörper (46) angeordnet ist, in Verbindung treten;
ein Gewindeabschnitt (43A), der in einer inneren Oberfläche der Befestigungsöffnung (43) gebildet ist, dazu fähig ist, mit einem anderen Schraubabschnitt (52A), der in einem Distalende der Fluidversorgungsquelle (52) gebildet ist, zu schrauben, wobei die Schraubrichtung der Fluidversorgungsquelle (52) und des Druckbedienabschnitts (48) dieselbe Richtung wie die Schraubrichtung des Druckbedienabschnitts (48) und des Ventilkörpers (46) ist; und
der Stopfenabschnitt (47) so gestaltet ist, dass er von dem Druckbedienabschnitt (48) druckbar ist, um sich innerhalb einer Basisendseite des Ventilkörpers (46) zu verformen, so dass eine zweite Durchtrittsöffnung (47A) des Stopfenabschnitts (47) zerdrückt wird.

2. Ballonkatheter (40) nach Anspruch 1, wobei
ein kleiner Ausnehmungsabschnitt (51) auf der Basisendseite des Ventilkörpers (46) angeordnet ist, und
der Stopfenabschnitt (47) dazu gestaltet ist, von dem Druckbedienabschnitt (48) gedrückt zu werden, um sich innerhalb des kleinen Ausnehmungsabschnitts (51) zu verformen.

3. Ballonkatheter (40) nach Anspruch 2, wobei
ein Ausnehmungsabschnitt mit großem Durchmesser (50) auf der Basisendseite des Ventilkörpers (46) gestaltet ist, und
der kleine Ausnehmungsabschnitt weiter zurückstehend von dem unteren Abschnitt des Ausnehmungsabschnitts mit großem Durchmesser (50) gebildet ist.

4. Ballonkatheter (40) nach Anspruch 1,
der ferner einen Regulierungsabschnitt (57) umfasst, der dazu ausgestaltet ist, eine Mitrotation des Ventilkörpers (46) beim Schrauben des Druckbedienabschnitts (48) in Bezug auf den Ventilkörper (46) zu regeln, der an dem Katheterschaft (3) angeordnet ist.

5. Ballonkatheter (40) nach Anspruch 1,
der ferner mit einem Bedienabschnitt (49) versehen ist, der den Ventilabschnitt (41) aufweist;
wobei ein Befestigungsabschnitt, der dazu ausgestaltet ist, den Bedienabschnitt lösbar an einem Endoskopbedienabschnitt, oder in der Nähe eines Endoskops, zu befestigen, an dem Bedienabschnitt (49) vorgesehen ist.

## Revendications

1. Cathéter à ballonnet (40) comprenant :
une tige de cathéter (3) ayant une flexibilité, avec une lumière (8) formée à l'intérieur ;
un ballonnet (5) qui est disposé sur le côté extrémité distale de la tige de cathéter (3) et en communication avec la lumière (8) ; et
une partie valve (41) dans laquelle est disposée une ouverture de liaison (42) qui est reliée au côté extrémité de base de la tige de cathéter (3), et une ouverture d'attache (43) qui est reliée à une source d'alimentation en fluide (52) configurée pour distribuer un fluide afin de dilater le ballonnet (5),
la partie valve (41) comportant un corps de valve (46) dans lequel est disposée une conduite (45) configurée pour permettre une communication entre la tige de cathéter (3) et la source d'alimentation en fluide (52), une partie obturateur (47) qui est disposée dans la conduite (45) et qui est configurée pour communiquer avec la conduite (45) ; et une partie d'actionnement de pression (48) qui est disposée dans une extrémité de base de la partie obturateur (47) et qui est configurée pour presser sur et déformer la partie obturateur (47) ;
l'ouverture d'attache (43) étant disposée à une partie extrémité d'un premier trou traversant (48A) dans la partie d'actionnement de pression (48) ;
l'ouverture de liaison (42) et l'ouverture d'attache (43) étant agencées pour communiquer par vissage de la partie d'actionnement de pression (48), dans laquelle la source d'alimentation en fluide (52) est vissée, dans le corps de valve (46) jusqu'à établir un contact avec la partie obturateur (47) disposée sur le corps de valve (46) ;
une partie filetage (43A) formée dans une surface interne de l'ouverture d'attache (43) étant capable d'être vissée avec une autre partie vis (52A) qui est formée dans une extrémité distale de la source d'alimentation en fluide (52), la direction de vissage de la source d'alimentation en fluide (52) et de la partie d'actionnement de pression (48) étant la même direction que la direction de vissage de la partie d'actionnement de pression (48) et du corps de valve (46) ; et
la partie obturateur (47) étant agencée pour être apte à être pressée par la partie d'actionnement de pression (48) pour se déformer élastiquement dans un côté extrémité de base du corps de valve (46) de telle sorte qu'un second trou traversant (47A) de la partie obturateur (47) est écrasé.

2. Cathéter à ballonnet (40) selon la revendication 1, dans lequel
une petite partie en renfoncement (51) est disposée sur le côté extrémité de base du corps de valve (46), et
la partie obturateur (47) est agencée pour être apte à être pressée par la partie d'actionnement de pression (48) pour se déformer élastiquement dans la petite partie en renfoncement (51).

3. Cathéter à ballonnet (40) selon la revendication 2, dans lequel
une partie en renfoncement de grand diamètre (50) est agencée sur le côté extrémité de base du corps de valve (46), et
la petite partie en renfoncement est formée davantage renfoncée à partir de la partie fond de la partie en renfoncement de grand diamètre (50).

4. Cathéter à ballonnet (40) selon la revendication 1,
comprenant en outre une partie de régulation (57) configurée pour réguler une co-rotation du corps de valve (46) pendant le vissage de la partie d'actionnement de pression (48) par rapport au corps de valve (46), et disposée sur la tige de cathéter (3).

5. Cathéter à ballonnet (40) selon la revendication 1,
comportant en outre une partie d'actionnement (49) qui a la partie valve (41) ;
une partie de fixation, configurée pour monter de manière détachable la partie d'actionnement sur une partie d'actionnement d'endoscope d'un endoscope ou au voisinage de celle-ci, étant située au niveau de la partie d'actionnement.
